# EUROPEAN PATENT APPLICATION

(11) **EP 2 839 796 A1**
(43) Date of publication of application: **25.02.2015**
(21) Application number: 14181066.3
(22) Date of filing: 14.08.2014
(51) Int. Cl.: A61B 17/11

(54) **Endovascular delivery system for magnetic compression vascular anastomosis**

(30) Priority: 23.08.2013 US 201361869114 P
(71) Applicant: Cook Medical Technologies LLC, Bloomington, IN 47404 (US)
(72) Inventor: Gittard, Shaun Davis, Winston-Salem, North Carolina 27101 (US); Martinez, Michelle D., Winston-Salem, North Carolina 27104 (US); Amatya, Devesh Man, Winston-Salem, North Carolina 27106 (US); Sigmon Jr., John C., Winston-Salem, North Carolina 27104 (US); Surti, Vihar Chandravadan, Winston-Salem, North Carolina 27104 (US); Haac, Jillian Faye, Winston-Salem, North Carolina 27106 (US); McLawhorn, Tyler Evans, Winston-Salem, North Carolina 27106 (US)
(74) Representative: Garratt, Peter Douglas

(57) **Abstract**

An endovascular delivery system for forming a magnetic compression vascular anastomosis includes a first compressing element and a first catheter configured for advancement into a first cardiovascular structure. In one configuration, the first compressing element is removably secured within the first catheter and, in another configuration, the first compressing element is released from the first catheter and positioned within the first cardiovascular structure. The endovascular delivery system also includes a second compressing element and a second catheter configured for advancement into a second cardiovascular structure. In one configuration, the second compressing element is removably secured within the second catheter and, in another configuration, the second compressing element is released from the second catheter and positioned within the second cardiovascular structure. The first and second compressing elements have an anastomosis forming configuration in which the compressing elements compress tissue of the first and second cardiovascular structures using magnetic force.

## Description

### Technical Field

The present disclosure relates generally to forming a magnetic compression vascular anastomosis and, more particularly, to an endovascular delivery system for delivering first and second compressing elements used for forming the magnetic compression vascular anastomosis.

### Background

A vascular shunt is a passageway that is created in the cardiovascular system, such as between blood vessels or other cardiovascular structures, to permit blood to flow around a site of injury or an occlusion. For example, a Glenn shunt is a surgical connection between the superior vena cava and the right pulmonary artery, allowing oxygen-poor blood to flow into the lungs. A Blalock-Taussig shunt is an artificial connection between the pulmonary artery and a systemic artery, such as the carotid artery or the subclavian artery, or a connection between the aortic and pulmonary arteries. According to another example, a surgically formed arteriovenous fistula may be created between an artery and a vein for hemodialysis treatments. According to a final example, a coronary artery bypass surgery restores blood flow to the heart by diverting the flow of blood around a section of blocked artery in the heart using a shunt.

Typically these shunts are formed during an invasive surgery. As should be appreciated, an invasive surgery typically involves large incisions, which enable a physician to work with their hands and various medical instruments inside a patient, while non-invasive surgery utilizes only small incisions and small instruments. Although there are advantages and disadvantages for both invasive and non-invasive surgeries, a non-invasive surgery may be preferred, unless the procedure dictates otherwise. In particular, a non-invasive surgery may offer a quicker recovery time and may be less risky when compared to an invasive surgery.

U.S. Patent No. 6,068,637 to Popov et al. (hereinafter "Popov") describes a method and device for performing a vascular anastomosis and is particularly presented as an alternative to surgically implanted coronary artery bypass grafts. In particular, a method and device are taught for surgically introducing a cutter catheter into a first hollow organ and a receiving catheter into a second hollow organ. Distal ends of the catheters include selectively activatable magnetic material that may be used to align the first and second hollow organs. After alignment, the organs may be secured together, using clips or glue, and a cutter material may remove sidewall portions of the first and second organs to form a passage therebetween. Although a procedure using the Popov device and method may be less invasive than some alternative coronary bypass procedures, the disclosed procedure nonetheless remains an invasive one.

The present disclosure is directed toward one or more of the problems or issues set forth above.

### Summary of the Disclosure

In one aspect, an endovascular delivery system for forming a magnetic compression vascular anastomosis includes a first magnet delivery system and a second magnet delivery system. The first magnet delivery system includes a first compressing element and a first delivery catheter configured for advancement into a first cardiovascular structure. The first magnet delivery system has a delivery configuration in which the first compressing element is removably secured within a lumen of the first delivery catheter, and a released configuration in which the first compressing element is released from the lumen of the first delivery catheter and positioned within the first cardiovascular structure. The second magnet delivery system includes a second compressing element and a second delivery catheter configured for advancement into a second cardiovascular structure. The second magnet delivery system has a delivery configuration in which the second compressing element is removably secured within a lumen of the second delivery catheter, and a released configuration in which the second compressing element is released from the lumen of the second delivery catheter and positioned within the second cardiovascular structure. The first compressing element and the second compressing element have an anastomosis forming configuration in which the first compressing element and the second compressing element compress tissue of the first cardiovascular structure and the second cardiovascular structure using a magnetic force between the first compressing element and the second compressing element.

In another aspect, a method of forming a magnetic compression vascular anastomosis using an endovascular delivery system is provided. The endovascular delivery system includes a first magnet delivery system including a first compressing element and a first delivery catheter configured for advancement into a first cardiovascular structure, and a second magnet delivery system including a second compressing element and a second delivery catheter configured for advancement into a second cardiovascular structure. The method includes a step of advancing a distal portion of the first delivery catheter to a delivery site within a first cardiovascular structure with the first magnet delivery system in a first delivery configuration in which the first compressing element is removably secured within a lumen of the first delivery catheter. Next, the first magnet delivery system is transitioned from the first delivery configuration to a first released configuration in which the first compressing element is released from the lumen of the first delivery catheter and positioned within the first cardiovascular structure. The method also includes a step of advancing a distal portion of the second delivery catheter to a delivery site within a second cardiovascular structure with the second magnet delivery system in a second delivery configuration in which the second compressing element is removably secured within a lumen of the second delivery catheter. Next, the second magnet delivery system is transitioned from the second delivery configuration to a second released configuration in which the second compressing element is released from the lumen of the second delivery catheter and positioned within the second cardiovascular structure. The method also includes steps of compressing tissue of the first cardiovascular structure and the second cardiovascular structure using a magnetic force between the first compressing element and the second compressing element, and allowing the compressed tissue to undergo necrosis and form the magnetic compression vascular anastomosis. The first transitioning step may include releasing a magnet assembly including a plurality of magnets into the first cardiovascular structure. The method may further include moving the magnet assembly into an assembled configuration after releasing the magnet assembly into the first cardiovascular structure by securing the plurality of magnets to one another magnetically or mechanically. The method may further include anchoring the first compressing element within the first cardiovascular structure by radially expanding a radially expandable prosthetic implant attached to the first compressing element within the first cardiovascular structure. The method may further include restricting movement of the first compressing element and the second compressing element after formation of the magnetic compression vascular anastomosis using a radial flange of each of the first compressing element and the second compressing element, wherein each radial flange is recessed relative to a vascular structure engagement face of a respective one of the first compressing element and the second compressing element. The compressing step may include forming a flap of tissue for anchoring the first compressing element and the second compressing element to at least one of the first cardiovascular structure and the second cardiovascular structure by utilizing a flap-forming magnet for one of the first compressing element and the second compressing element, wherein a vascular structure engagement face of the flap-forming magnet includes a recessed area that is partially surrounded by a non-recessed area and extends to an edge of the vascular structure engagement face. The method may further include incising the first cardiovascular structure and the second cardiovascular structure to remove the flap of tissue, the first compressing element, and the second compressing element. Transitioning the first magnet delivery system from the first delivery configuration to the first released configuration may include moving the first compressing element out of magnetic engagement with a portion of the first delivery catheter. The method may further include expediting the formation of the magnetic compression vascular anastomosis using ablation. The method may further include fusing the compressed tissue to define a perimeter of the magnetic compression vascular anastomosis; and removing tissue within the perimeter to form the magnetic compression vascular anastomosis.

### Brief Description of the Drawings

Fig. 1 is a sectioned side diagrammatic view of an endovascular delivery system, according to one embodiment of the present disclosure;
Fig. 2 is a perspective view of an exemplary elliptically shaped magnet configured for use with the endovascular delivery system of Fig. 1;
Fig. 3 is a perspective view of a magnet assembly including a plurality of magnets, shown in a delivery configuration and configured for use with the endovascular delivery system of Fig. 1;
Fig. 4 is a perspective view of the magnet assembly of Fig. 3, shown in an assembled configuration and configured for use with the endovascular delivery system of Fig. 1;
Fig. 5 is a side diagrammatic view of exemplary stacks of magnets configured for use with the endovascular delivery system of Fig. 1;
Fig. 6 is a side diagrammatic view of exemplary magnets having protrusions, which are configured for use with the endovascular delivery system of Fig. 1;
Fig. 7 is a side diagrammatic view of exemplary magnets having respective peak and valley profiles configured for use with the endovascular delivery system of Fig. 1;
Fig. 8 is a perspective view of a magnet having a hole therethrough for receiving a wire guide and being configured for use with the endovascular delivery system of Fig. 1;
Fig. 9 is a side diagrammatic view of a pair of magnets having RFID chips, which may be used with the endovascular delivery system of Fig. 1 and configured for signaling formation of a magnetic compression vascular anastomosis;
Fig. 10 is a perspective view of an anchored magnet attached to a radially expandable stent and configured for use with the endovascular delivery system of Fig. 1;
Fig. 11 is a perspective view of a pair of coapted magnets, each including a radial flange and configured for use with the endovascular delivery system of Fig. 1;
Fig. 12 is a perspective view of one embodiment of a flap-forming magnet configured for use with the endovascular delivery system of Fig. 1;
Fig. 13 is a perspective view of a magnet frame according to another embodiment of a flap-forming magnet configured for use with the endovascular delivery system of Fig. 1;
Fig. 14 is a side diagrammatic view of first and second cardiovascular structures of a patient at one stage of a magnetic compression vascular anastomosis procedure using the endovascular delivery system of Fig. 1;
Fig. 15 is a side diagrammatic view of the first and second cardiovascular structures at another stage of a magnetic compression vascular anastomosis procedure using the endovascular delivery system of Fig. 1;
Fig. 16 is a side diagrammatic view of the first and second cardiovascular structures at another stage of a magnetic compression vascular anastomosis procedure using the endovascular delivery system of Fig. 1;
Fig. 17 is a side diagrammatic view of the first and second cardiovascular structures at another stage of a magnetic compression vascular anastomosis procedure using the endovascular delivery system of Fig. 1;
Fig. 18 is a side diagrammatic view of the first and second cardiovascular structures at another stage of a magnetic compression vascular anastomosis procedure, depicting an exemplary anchoring mechanism;
Fig. 19 is a side diagrammatic view of the first and second cardiovascular structures at another stage of a magnetic compression vascular anastomosis procedure, depicting another exemplary anchoring mechanism;
Fig. 20 is a side diagrammatic view of the first and second cardiovascular structures at another stage of a magnetic compression vascular anastomosis procedure, depicting yet another exemplary anchoring mechanism;
Fig. 21 is a side diagrammatic view of the first and second cardiovascular structures at another stage of a magnetic compression vascular anastomosis procedure, depicting an exemplary retrieval catheter;
Fig. 22 is a partially sectioned side diagrammatic view of an endovascular delivery system according to another aspect of the present disclosure;
Fig. 23 is a perspective view of a pair of compressing elements according to another aspect of the present disclosure;
Fig. 24 is a front section view of the pair of compressing elements of Fig. 23 in a mated configuration;
Fig. 25 is a side section view of a pair of compressing elements according to still another aspect of the present disclosure; and
Fig. 26 is a perspective view of a second compressing element from the pair of compressing elements of Fig. 25.

### Detailed Description

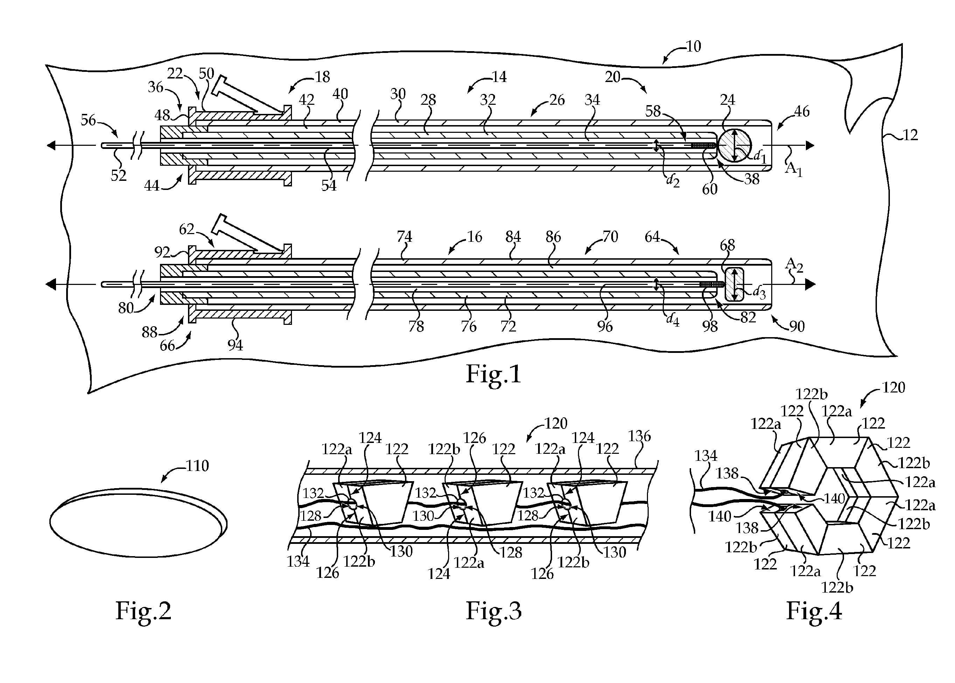

Referring to Fig. 1, there is shown an endovascular delivery system 10 for forming a magnetic compression vascular anastomosis, according to one embodiment of the present disclosure. The endovascular delivery system 10 may include a number of components, which may be provided within a sterile, tear open package 12, as is known in the art. In performing a magnetic compression vascular anastomosis, some or all of the components of the endovascular delivery system 10 may be used, depending upon the specifics of the procedure to be performed. As should be appreciated, however, the components shown in Fig. 1 might be separately packaged and/or the endovascular delivery system 10 might also include components in addition to those shown, including components routinely used in percutaneous vascular procedures.

As shown, the endovascular delivery system 10 may include a first magnet delivery system 14 and a second magnet delivery system 16. The first magnet delivery system 14 has a proximal end 18 and a distal end 20. As shown, a handle assembly 22, which may include relatively rigid components made from medical grade materials, may be disposed at the proximal end 18. In the present disclosure, "proximal" will be used to refer to the end of a component or feature that is closest to a clinician, while "distal" is used to refer to a component or feature that is farthest away from the clinician. Such meanings are consistent with conventional use of the terms and, as such, should be understood by those skilled in the art.

The first magnet delivery system 14 includes a first compressing element 24, which may include a magnet or a ferromagnetic material, and a first delivery catheter 26 configured for advancement into a cardiovascular structure. According to the exemplary embodiment, the first delivery catheter 26 may include an outer tube 28 telescopically received within an outer sheath, or catheter, 30. The outer tube 28 may have an elongate tubular body 32 defining a lumen 34 extending from an open proximal end 36 to an open distal end 38. The outer sheath 30 may similarly include an elongate tubular body 40 defining a lumen 42 extending from an open proximal end 44 to an open distal end 46. Each of the outer tube 28 and the outer sheath 30 may be made from any common medical tube material, such as, for example, a plastic, rubber, silicone, or Teflon material, and may exhibit both firmness and flexibility. Typically, each of the outer tube 28 and the outer sheath 30 may range in length from several inches to several feet long, and may have a wall diameter that is orders of magnitude smaller than its length.

The open proximal end 36 of the outer tube 28 may include an inner handle 48, or other similar component, which may define a portion of the handle assembly 22. Another portion of the handle assembly 22 may be defined by an outer handle 50 disposed at the open proximal end 44 of the outer sheath 30. According to the exemplary embodiment, the handle assembly 22 may be configured such that the inner handle 48 and the outer handle 50 are axially movable relative to one another along a longitudinal axis A₁ of the first delivery catheter 26. For example, while maintaining a relatively stationary position of the outer handle 50, a clinician may manipulate the inner handle 48 to insert and withdrawal the outer tube 28 relative to the outer sheath 30. Although a specific handle assembly 22 is shown, it should be appreciated that alternative components, including hubs, may be used for grasping and manipulating portions of the first magnet delivery system 14.

The first magnet delivery system 14 may also include an inner tube 52 having an elongate body 54, which may be a solid structure, as shown, or may be hollow, with a tubular body defining an internal lumen. According to an exemplary embodiment, the elongate body 54 may be formed from a plastic or metal, or other commonly selected material, to provide a desired combination of stiffness and flexibility. For example, a certain degree of stiffness may be required for pushability and trackability, while a certain degree of flexibility may be required for improving navigation. It should be appreciated that the stiffness of the elongate body 54 may be consistent along a longitudinal axis of the body or may vary, depending on the specifics of the procedure to be performed and the performance characteristics desired. The same holds true for materials and configurations of the first delivery catheter 26.

As shown, the inner tube 52 may be telescopically received within the outer tube 28 and, according to some embodiments, may have a length that is greater than the lengths of the outer tube 28 and the outer sheath 30. For example, it may be desirable for a clinician to grasp and manipulate a proximal end 56 of the inner tube 52 to reposition a distal end 58 of the inner tube 52 relative to the open distal end 38 of the outer tube 28 and the open distal end 46 of the outer sheath 30. According to the exemplary embodiment, the inner tube 52 may include a magnetized or magnetically attractive distal tip 60 that may require repositioning relative to the outer tube 28, the outer sheath 30, and the first compressing element 24.

In particular, the magnetized, or magnetically attractive, distal tip 60 and the first compressing element 24 may be configured such that a magnetic force between the magnetized distal tip 60 and the first compressing element 24 may removably secure the first compressing element 24 with respect to the first delivery catheter 26. Alternative arrangements for removably securing the first compressing element 24 with respect to the first delivery catheter 26 are also contemplated. For example, the magnetized distal tip 60 may be replaced with an electromagnetic tip that may be activated or deactivated or a tip made from a ferrous material. Although the first compressing element 24 may be a magnet or may be made from a ferromagnetic material, it will also be referred to herein as a first magnet.

Fig. 1 depicts a delivery configuration of the first magnet delivery system 14 in which the outer tube 28 is telescopically received within the outer sheath 30, and the open distal end 38 of the outer tube 28 is positioned proximally relative to the open distal end 46 of the outer sheath 30. The first magnet 24 is positioned within the outer sheath 30 and between the open distal end 38 of the outer tube 28 and the open distal end 46 of the outer sheath 30. The first magnet 24 may be oriented such that a central axis of the magnet 24 is substantially aligned with the longitudinal axis *A*₁ of the delivery catheter 26, as shown, or may have alternative orientations within the outer sheath 30. Importantly, according to the exemplary embodiment, a diameter *d*₁ of the first magnet 24 is greater than an inner diameter *d*₂ of the outer tube 28 and, thus, may not be received within the outer tube 28. For alternative magnet embodiments having different shapes or configurations, the alternative magnets may likewise be dimensioned to preclude receipt of the alternative magnet within the lumen 34 of the outer tube 28. Also according to the delivery configuration, the inner tube 52 is received within the outer tube 28 and has the magnetized distal tip 60 positioned in close enough proximity to the first magnet 24 to permit a magnetic force between the magnetized distal tip 60 and the first magnet 24 to removably secure the first magnet 24 with respect to the first delivery catheter 26.

Lengths of each of the outer tube 28, the outer sheath 30, and the inner tube 52 may be selected based on the desired manipulation of the first delivery catheter 26, particularly to transition the first magnet delivery system 14 from the delivery configuration, which is shown, to a released configuration, which will be discussed below. For example, it may be desirable for the inner tube 52 to have a length that extends proximally beyond the outer tube 28 an amount sufficient to pull or retract the inner tube 52 away from the first magnet 24 while pushing or maintaining stationary the first magnet 24 with the open distal end 38 of the outer tube 28. By moving the magnetized distal tip 60 and first magnet 24 away from one another such that the magnetic force between the magnetized distal tip 60 and the first magnet 24 no longer pulls them together, the first magnet 24 may thereafter be released or deployed. According to another example, it may be desirable for the outer tube 28 to have a length that extends proximally beyond the outer sheath 30 an amount sufficient to assist in moving the magnetized distal tip 60 of the inner tube 52 and the first magnet 24 away from one another and/or to push or deploy the first magnet 24 distally beyond the open distal end 46 of the outer sheath 30. According to alternative embodiments, for example, an electromagnetic tip of the inner tube 52 may be deactivated to release the first magnet 24.

Although not required, the exemplary embodiment of the second magnet delivery system 16 of the endovascular delivery system 10 may be similar to the above-described embodiment of the first magnet delivery system 14. The second magnet delivery system 16 generally has a proximal end 62 and a distal end 64, with a handle assembly 66 disposed at the proximal end 62. The second magnet delivery system 16 is sized and configured for advancement into a cardiovascular structure and generally includes a second compressing element 68, which is also referenced herein as a second magnet, and a second delivery catheter 70. Although any number of tubes may be utilized, including a single tube, the second delivery catheter 70 may also include an outer tube 72 telescopically received within an outer sheath 74. The outer tube 72 may have an elongate tubular body 76 defining a lumen 78 extending from an open proximal end 80 to an open distal end 82. The outer sheath 74 may similarly include an elongate tubular body 84 defining a lumen 86 extending from an open proximal end 88 to an open distal end 90. Materials and dimensions for the outer tube 72 and the outer sheath 74 may be similar to those described above with respect to the outer tube 28 and the outer sheath 30 of the first delivery catheter 26.

The open proximal end 80 of the outer tube 72 may include an inner handle 92, while the open proximal end 88 of the outer sheath 74 may include an outer handle 94. The inner handle 92 and the outer handle 94 may be used, as described above, to move the second delivery catheter 70 into different configurations. Although a handle assembly is not required, alternative handles or handle assemblies are also contemplated for achieving the desired movements. The second magnet delivery system 16 may also include an inner tube 96, similar to the inner tube 52 of the first magnet delivery system 14, telescopically received within the outer tube 72. The inner tube 96 may include a magnetized or magnetically attractive distal tip 98 that may require repositioning relative to the outer tube 72, the outer sheath 74, and the second magnet 68, as described above.

Fig. 1 depicts a delivery configuration of the second magnet delivery system 16 in which the outer tube 72 is telescopically received within the outer sheath 74, and the open distal end 82 of the outer tube 72 is positioned proximally relative to the open distal end 90 of the outer sheath 74. The second magnet 68 is positioned within the outer sheath 74 and between the open distal end 82 of the outer tube 72 and the open distal end 82 of the outer sheath 74. The second magnet 68 may be oriented such that a central axis of the magnet 24 is substantially perpendicular to the longitudinal axis *A*₂ of the delivery catheter 70, as shown, or may have alternative orientations within the outer sheath 74. The orientations of the first and second magnets 24 and 68 during delivery may be the same or different, depending on the specifics of the procedure being performed. Importantly, according to the exemplary embodiment, a diameter *d*₃ of the second magnet 68 is greater than an inner diameter *d**₄ of the outer tube 72 and, thus, may not be received within the outer tube 72. The inner tube 96 is received within the outer tube 72 and has the magnetized distal tip 98 positioned in close enough proximity to the second magnet 68 to permit a magnetic force between the magnetized distal tip 98 and the second magnet 68 to removably secure the second magnet 68 with respect to the second delivery catheter 70. As stated above, alternative arrangements for removably securing the second magnet 68 with respect to the second delivery catheter 70 are also contemplated.

As shown in Fig. 1, each of the first magnet 24 and the second magnet 68 may have a circular cross section and may be magnetized through the thickness of the respective magnet 24, 68. Alternatively, only one of the first and second magnets 24 and 68 may be magnetized, while the other of the first and second magnets 24 and 68 may be made from a ferrous material, such as a material containing iron or nickel, which may include a bulk solid, granules, powder, gel, or liquid. Although the first and second magnets 24 and 68 are shown having a similar shape and size, the first and second magnets 24 and 68 may differ from one another in at least these aspects. Further, the first and second magnets 24 and 68 may be any of a variety of shapes and sizes. Exemplary cross sections may include circular, elliptical, rectangular, rounded rectangle, triangular, and trapezoidal, to name a few.

For example, an elliptically shaped magnet 110, having an elliptical cross section, as shown in Fig. 2, may be substituted for one or both of the first and second magnets 24 and 68. The elliptically shaped magnet 110 may provide a greater surface area than a magnet having a circular shape and, therefore, may be used to create a magnetic compression vascular anastomosis having a correspondingly bigger opening. In addition, the elliptically shaped magnet 110 may offer an increased surface area while still maintaining a low enough profile for receipt into one of the first and second delivery catheters 26 and 70, as described above. As will be described below, the first and second magnets 24 and 68 are configured such that a magnetic force between the first and second magnets 24 and 68 may compress tissue therebetween to ultimately form a magnetic compression vascular anastomosis.

Turning now to Fig. 3, at least one of the first magnet 24 and the second magnet 68 may be a magnet assembly 120 including a plurality of magnets 122. As shown, each of the magnets 122 may include halves 122a and 122b magnetized such that opposing faces 124 and 126 are drawn together. For example, halves 122a may have a polarity that is opposite the polarity of halves 122b. Each of the halves 122a and 122b may include a central groove 128 and 130 positioned such that, when the halves 122a and 122b are magnetically joined, the central grooves 128 and 130 of each of the halves 122a and 122b form a hole 132 through the magnet 122 for receiving a string 134, or other similar mechanical retention device. Although only a few magnets 122 are depicted, any number of magnets 122 may be provided. The magnet assembly 120 is shown in a delivery configuration in Fig. 3 in which the magnets 122 are sequentially disposed, such as within a delivery catheter 136. Although the magnets 122 are shown having a trapezoidal shape, it should be appreciated that alternative shapes, including pie slice shapes, may be used.

Regardless of whether or not the magnets 122 include halves 122a and 122b, the magnets 122 may be configured to ultimately form an assembled configuration of the magnet assembly 120, an example of which is shown in Fig. 4. For example, once the magnet assembly 120 is released into a cardiovascular structure, ends of the string 134 may be manipulated to draw the magnets 122 into an assembled annular shape, as shown. Alternatively, or additionally, the magnet assembly 120 may be configured such that the magnets 122 are magnetically drawn into the position shown. For example, the magnets 122 may be positioned such that halves 122a are positioned adjacent halves 122b since their polarities may be opposite. Accordingly, the magnets 122 may "self-assemble" when released from the delivery catheter 136. In particular, ends 138 of halves 122a may be magnetically drawn to adjacent ends 140 of halves 122b. The magnet assembly 120 should also be configured such that, when deployed within a cardiovascular structure, the magnet assembly 120 will coapt with a magnet or magnet assembly of another cardiovascular structure to form a magnetic compression vascular anastomosis.

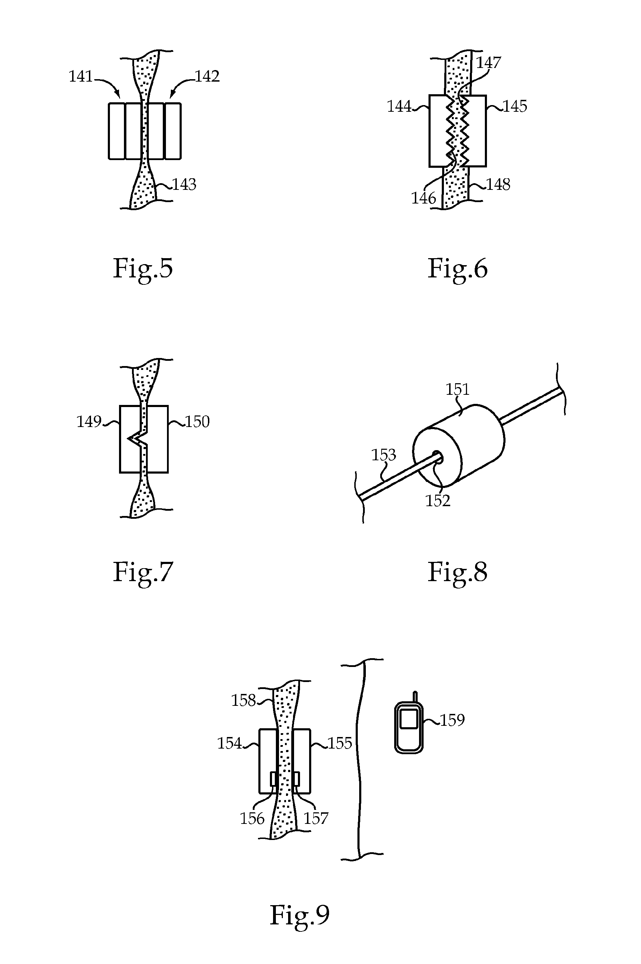

One or both of the first and second magnets 24 and 68 may be configured or adapted to increase the compressive force between the magnets 24 and 68 and/or accelerate tissue necrosis during the formation of a magnetic compression vascular anastomosis. For example, as shown in Fig. 5, a first stack of magnets 141 may be delivered to one cardiovascular structure, while a second stack of magnets 142 may be delivered to another cardiovascular structure. The first and second magnet stacks, or assemblies, 141 and 142 may compress tissue 143 therebetween, as described herein, to form a magnetic compression vascular anastomosis. A greater compressive force may be provided using magnet stacks 141 and 142, which each include more than one magnet, as compared to using single magnets.

According to another arrangement, each of the first magnet 24 and the second magnet 68 may include an irregular surface profile configured to engage tissue of a respective one of a first cardiovascular structure and a second cardiovascular structure. For example, as shown in Fig. 6, magnets 144 and 145 having respective protrusions 146 and 147 extending from tissue engaging faces of the magnets 144 and 145 may be substituted for first and second magnets 24 and 68 to increase pressure and/or damage tissue 148 compressed therebetween to accelerate tissue necrosis. Turning to Fig. 7, magnets 149 and 150 having mating profiles, such as the peak and valley profiles that are shown, may alternatively be used to accelerate tissue necrosis. Although specific irregular surface profiles are shown, it should be appreciated that any of a variety of irregular, or non-planar, surface profiles may be used to shorten the time required to form a magnetic compression vascular anastomosis.

Turning now to Fig. 8, a magnet 151 having a hole 152 therethrough may be substituted for one or both of the magnets 24 and 68, and may be configured for use with the endovascular delivery system 10. For example, the magnet 151 may be threaded over a wire guide 153 during a delivery procedure using a delivery system similar to the endovascular delivery system 10 described herein. For example, a pusher device may be used to distally advance the magnet 151 over the wire guide 153 in a known fashion.

As shown in Fig. 9, magnets 154 and 155 having radio-frequency identification (RFID) chips 156 and 157 may be delivered to adjacent cardiovascular structures. The RFID chips 156 and 157 may be configured such that when tissue 158 compressed by magnets 154 and 155 undergoes necrosis and the magnets 154 and 155 contact one another, the RFID chips 156 and 157 define a circuit and transmit a signal to an RFID reader 159 positioned outside the body. As such, an indication of formation of a magnetic compression vascular anastomosis may be provided.

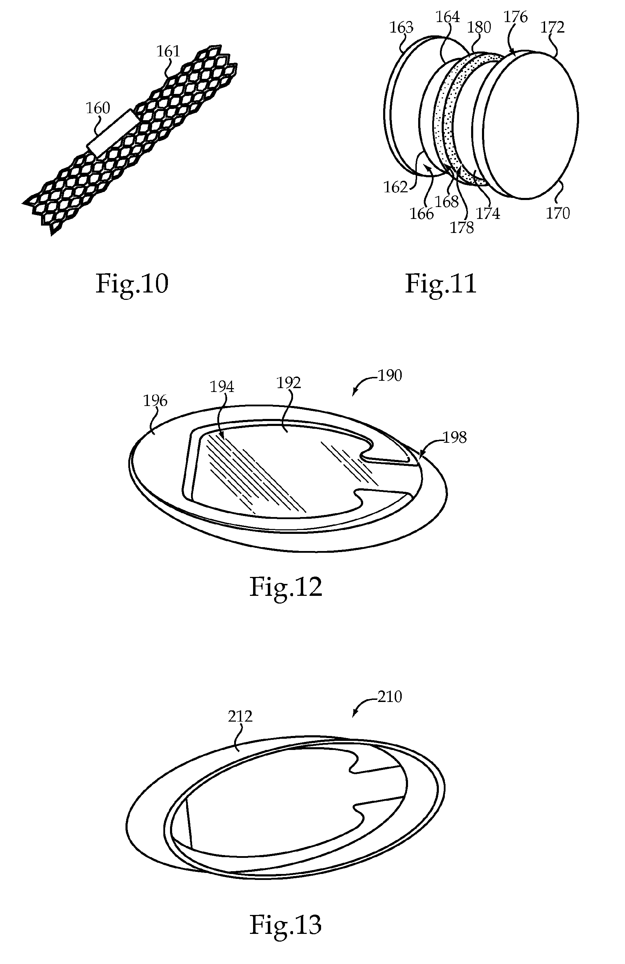

One or both of the first magnet 24 and second magnet 68, which may each include any of a variety of shapes, sizes, and configurations, may include an anchoring mechanism. An anchoring mechanism may be used to anchor the first and second magnets 24 and 68 after formation of the magnetic compression vascular anastomosis to reduce the risk of an embolism. Additionally, or alternatively, an anchoring mechanism may be provided to maintain a desired magnet positioning during any stage of a magnetic compression vascular anastomosis procedure. According to a first example, as shown in Fig. 10, one or both of the first magnet 24 and the second magnet 68 may be an anchored magnet 160 attached to a radially expandable prosthetic implant, such as a stent, 161, which is configured to anchor the anchored magnet 160 within a cardiovascular structure. Radially expandable stents, such as stent or other device 161, are known and may be expanded using a balloon, or other known device, positioned at a distal portion of a delivery catheter. Alternatively, the radially expanding stent 161 may be made from a resilient or shape memory material, such as, for example, nitinol, that is capable of self-expanding from a compressed state to an expanded state without the application of a radial force on the stent 161. As such, the stent 161 may also be referred to as a "self-expanding" stent. Although a stent 161 is shown, it should be appreciated that an alterative prosthetic implant, such as a stent graft or venous filter may alternatively be used.

During a magnetic compression vascular anastomosis procedure, for example, the anchored magnet 160 and stent 161 may be delivered and deployed using the first magnet delivery device 14 described above. For example, a magnetic force may be used to removably secure the anchored magnet 160 and stent 161 with respect to the first delivery catheter 26. Respective components may be later repositioned to remove that magnetic force, and/or the outer tube 28 may be used, to deploy the radially expandable stent 161 and, thus, the anchored magnet 160. Although the anchored magnet 160 is shown as directly attached to a frame or body of the stent 161, it is contemplated that the anchored magnet 160 may be tethered to the stent 161, or otherwise indirectly attached thereto.

As shown in Fig. 11, a first magnet 162 may include a radial flange 163 extending from a base portion 164 of the first magnet 162. The radial flange 163 may be continuous or discontinuous about the base portion 164 of the first magnet 162 and may have a recessed face 166 that is recessed relative to a vascular structure engagement face 168 of the first magnet 162. Similarly, a second magnet 170 may include a radial flange 172 extending radially from a base portion 174 of the second magnet 170. The radial flange 172, which may also be continuous or discontinuous, may have a recessed face 176 that is recessed relative to a vascular structure engagement face 178 of the second magnet 170. During formation of a magnetic compression vascular anastomosis, a magnetic force between the first magnet 162 and the second magnet 170 compresses tissue 180 between the vascular structure engagement faces 168 and 178 to form a magnetic compression vascular anastomosis. Once the tissue 180 undergoes necrosis, the first and second magnets 162 and 170, which might otherwise be free to flow downstream within a respective cardiovascular structure, may be anchored or restricted using the radial flanges 163 and 172.

In Fig. 12, another exemplary anchoring mechanism is shown. For example, at least one of the first magnet 24 and second magnet 68 may be a flap-forming magnet 190 having recessed area 192 along a vascular structure engagement face 194 of the flap-forming magnet 190. The recessed area 192 is partially surrounded by a non-recessed area 196 of the vascular structure engagement face 194 and extends to an edge 198 of the vascular structure engagement face 194. When the flap-forming magnet 190 is magnetically coupled with a magnet having a substantially planar vascular structure engagement face to compress tissue therebetween, tissue corresponding to the non-recessed area 196 will undergo necrosis while leaving the tissue corresponding to, or aligned with, the recessed area 192 intact. As such, a pair of magnets, including the flap-forming magnet 190, will be tethered to the compressed tissue, i.e., vascular structures, by a newly formed flap of tissue.

As an alternative, a flap-forming magnet 210 may include a magnet body, such as the elliptically shaped magnet 110 shown in Fig. 2, supported within a magnet frame 212, as shown in Fig. 13. The magnet body, which may include magnet 110 of Fig. 2, may define a recessed area, similar to the recessed area 192 of Fig. 12, while the magnet frame 212 forms a non-recessed area, similar to the non-recessed area 196 of Fig. 12. As should be appreciated, different configurations may be selected for various reasons, including manufacturing preferences.

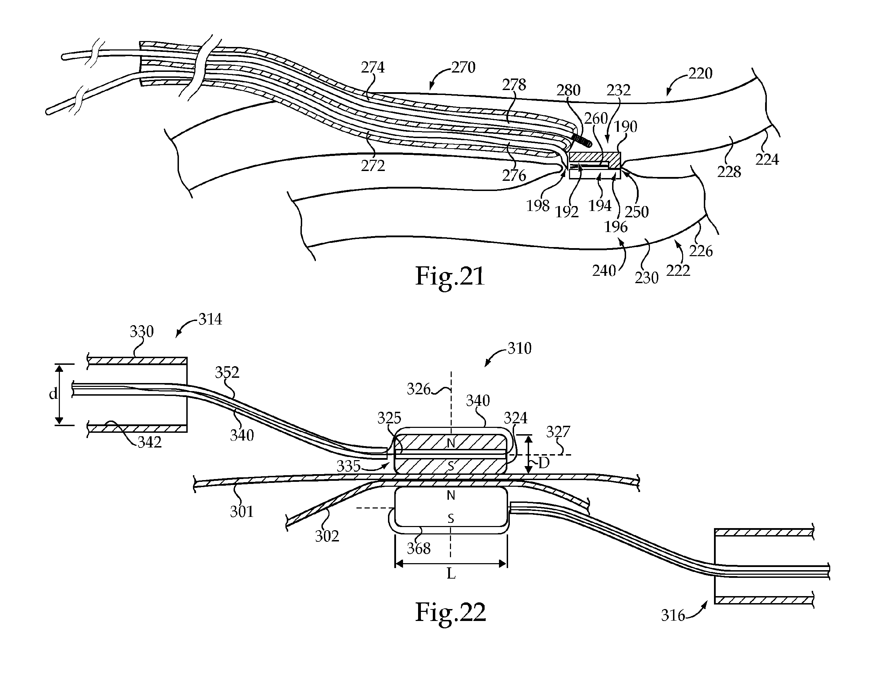

Referring now to Fig. 22, and endovascular delivery system 310 includes a first magnet delivery system 314 and a second magnet delivery system 316 that are substantially identical and both shown in a pre-release configuration. The pre-release configuration is characterized in the case of the first magnetic delivery system 14 by a first compressing element 324 being positioned outside of a lumen 342 of a first delivery catheter 330, and mechanically connected with a mechanical connection 335 to an inner tube 352 that is telescopically received in the first delivery catheter 330. The first compressing element or magnet 324 and the second compressing element/magnet 368 may have a generally cylindrical or tubular shape with a length L to diameter D ratio greater than one. In addition, the length L may be greater than the diameter d of lumen 342. The first magnet 324 includes a hole 325 that facilitates the mechanical connection 335 via a suture 340 that extends inside of inner tube 352 and loops through hole 325 so that the operator can insure that both the first magnet 324 and the second magnet 368 are at the desired locations with respect to tissue walls 301 and 302 before disconnecting the mechanical connection 335 by pulling one end of suture 340 through and out of hole 325. In this embodiment, the two cylindrically shaped magnets 324 and 368 may have their magnetic pole axis 326 oriented perpendicular to their respective long axes 327 to focus the compressive force onto tissue walls 301, 302 along a tangent line. When the magnets 324, 368 are magnetized with this orientation, they will preferentially mate along their side instead of on their end faces. This allows for a greater compressive force while still having a smaller delivery system. In addition, tissue growth may be encouraged in the areas of lesser compression that surround the line of maximum compression between the two magnets 324, 368. The pre-release configuration illustrated in Fig. 22 allows the user to potentially return to a delivery configuration if one or both of the magnets 324, 368 finds difficulty in being properly placed. As discussed earlier, the delivery configuration would have magnet 324 still located within lumen 342 by telescopically retracting inner tube 352 in a proximal direction.

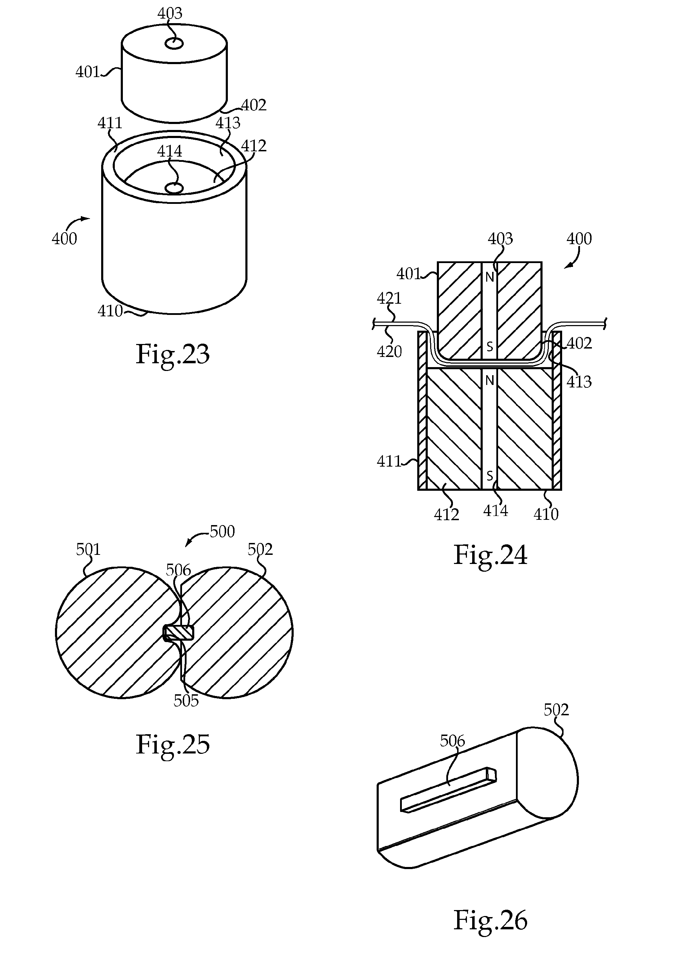

Referring now to Figs. 23 and 24, a pair of compressing elements 400 include a first compressing element/magnet 401 that defines a hole 403 and includes a male surface feature 402. A second compressing element 410 includes a magnet with an attached non-magnetic jacket 411 that partially defines a female surface feature 413 sized to receive the male surface feature 402 of the first compressing element 401. Second compressing element 410 may also include a hole 414 to facilitate delivery as discussed earlier. Fig. 24 is of interest for showing the male surface feature 402 of the first compressing element 401 received in the female surface 413 of the second compressing element 410, trapping tissue walls 420 and 421 therebetween.

Referring now to Figs. 25 and 26, a pair of compressing elements 500 according to still another aspect of the present disclosure include a first elongate compressing element 501 with a female surface feature 505 sized to receive a male surface feature 506 of a second compressing element 502. The mating of the male surface feature 506 with the female surface feature 505 may help to concentrate the compression force on the tissue (not shown) trapped between the two compressing elements 501, 502. In the illustrated embodiment, the male surface feature 506 may include a protrusion made from a non-magnetic material that is attached to the underlying second magnet 502 as shown.

Those skilled in the art will appreciate that the magnets according to the present disclosure may be made form a variety of materials and types such as neodymium-iron-boron, samarium-cobalt, Alnico, or any suitable ferrite could also be used. Depending upon the application, the compressing elements may be made from, or coated with, a hemocompatible material, such as parylene, gold, nickel, PTFE, silicon, ePTFE, polyester or an other suitable material. On the other hand, in some applications it might be desirable to have some or all of the surface features have an irritant or surface material that may induce adhesion of the two tissues or vessels to each other. For instance, this may include a roughened surface, dissimilar metal surfaces to induce galvanic corrosion, or possibly tissue irritating materials known in the art. Those skilled in the art will appreciate that the compressive elements according to the present disclosure may include holes, protrusions, grooves tails or any other feature that enable the compressive element to be grasped or held for delivery and retrieval instead of relying upon magnetic detachment for delivery as discussed with regard to the earlier embodiments.

Referring again to Fig. 22, the inner member 352 may have sufficient stiffness to help push magnet 324 out of delivery catheter 330, but have a sufficiently flexible tip that the bending force of the member would not be counteracting the attractive forces of the compressive elements 324, 368. Preferably, but not necessarily, most or all of the delivery system may be made from non-magnetic elements in order to prevent the implant magnets from being attracted to unwanted sections of the delivery system. For instance, it may not be desirable to use stainless steel coil springs or catheters reinforced with braided stainless steel. Those skilled in the art will appreciate that the compressing elements or magnets could be held to a delivery member by any suitable means such as a grasping element, a snare, forceps, a balloon, magnetic force, or suture that permits controlled release once the compressive elements are mated at their desired implant location.

### Industrial Applicability

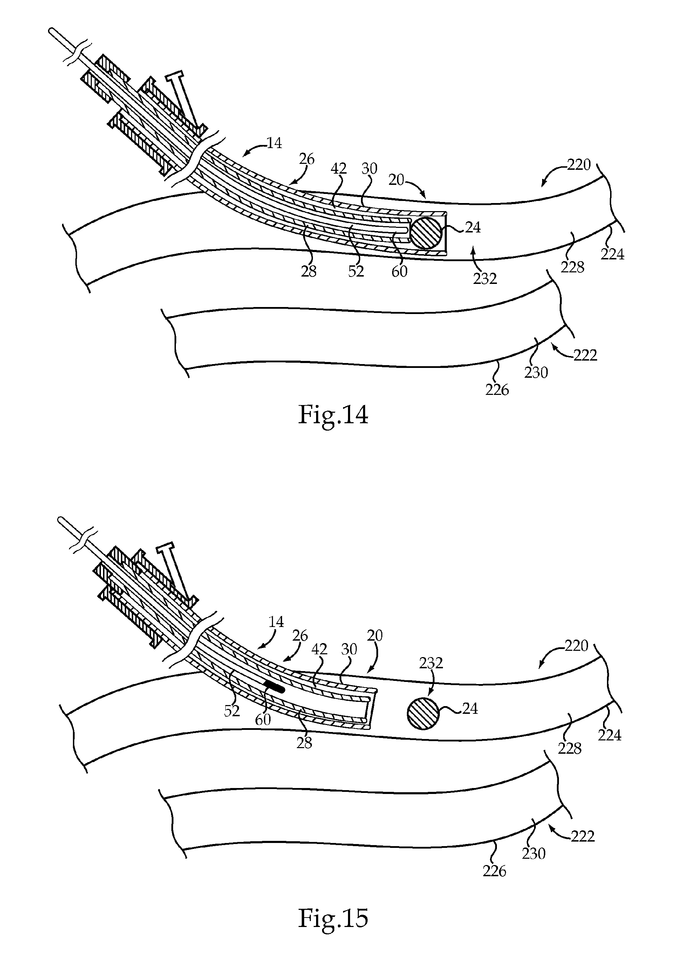

The endovascular delivery system 10 described herein may be used to form a magnetic compression vascular anastomosis. In particular, a percutaneous vascular procedure for forming the magnetic compression vascular anastomosis using the endovascular delivery system 10 will now be described with reference to a first cardiovascular structure 220 and a second cardiovascular structure 222 of a patient, as shown in Fig. 14. Each of the cardiovascular structures 220 and 222 may include a respective vessel wall 224, 226, or other cardiovascular structure wall, defining a respective lumen 228, 230.

A clinician may first position a needle, or introducer, through the skin of a patient to gain access to the first cardiovascular structure 220 in a known manner. For example, access may be gained through femoral, jugular, radial, apical, aortic, or carotid arteries. At a next stage of the procedure, and with the use of conventional radiological techniques, a clinician may insert a conventional wire guide through a tube of the introducer and into the first cardiovascular structure 220. The distal portion 20 of the first magnet delivery system 14 may be inserted through the introducer and over the wire guide. In particular, and with the first magnet delivery system 14 positioned in the delivery configuration of Fig. 1, the distal portion 20 of the first delivery catheter 26 may be advanced to a delivery site 232 within the first cardiovascular structure 220.

Next, the first magnet delivery system 14 may be transitioned from the first delivery configuration of Fig. 1 to a first released configuration, as shown in Fig. 15, in which the first magnet 24 is released from the lumen 42 of the outer sheath 30 and positioned within the first cardiovascular structure 220. For example, this transitioning step may include moving the first magnet 24 out of magnetic engagement with the magnetized distal tip 60 of the inner tube 52 and/or moving the outer tube 28 relative to the outer sheath 30 and/or inner tube 52, as described above. As indicated above, alternative means for releasing or deploying the first magnet 24 are also contemplated.

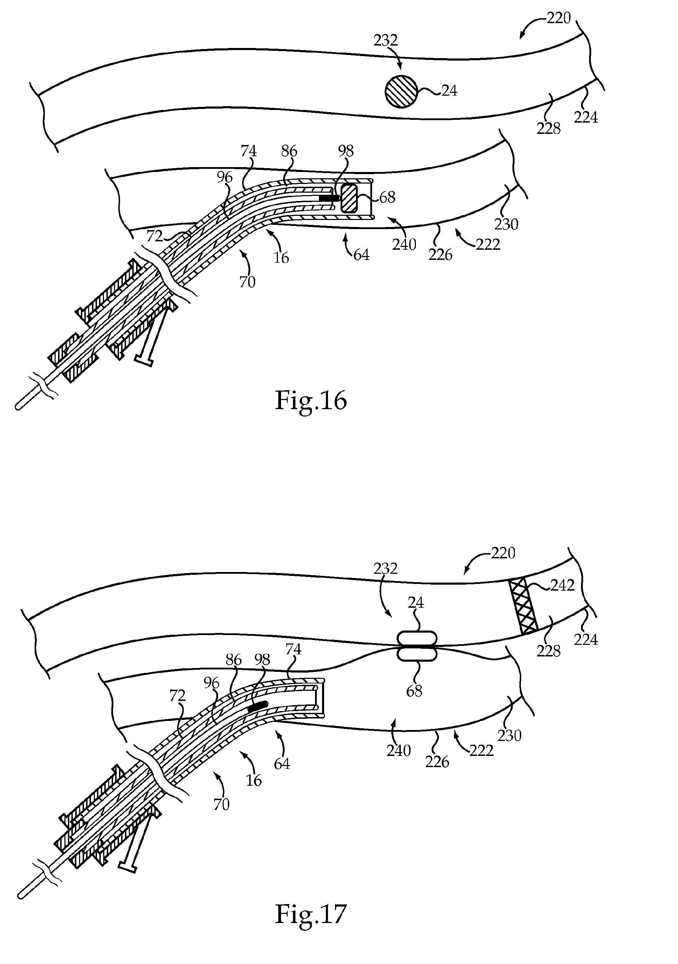

At another stage of the procedure, the clinician may again position a needle, or introducer, through the skin of the patient to gain access to the second cardiovascular structure 222. The clinician may then insert a conventional wire guide through a tube of the introducer and into the second cardiovascular structure 222. The distal portion 64 of the second magnet delivery system 16 may be inserted through the introducer and over the wire guide. In particular, and with the second magnet delivery system 16 positioned in the delivery configuration of Fig. 1, the distal portion 64 of the second delivery catheter 70 may be advanced to a delivery site 240 within the second cardiovascular structure 222, as shown in Fig. 16. It should be appreciated that the delivery sites 232 and 240 within the first and second cardiovascular structures 220 and 222 may correspond to a desired location of a shunt between the first and second cardiovascular structures 220 and 222. It should also be appreciated that the first and second cardiovascular structures 220 and 222, at the first and second delivery sites 232 and 240, should be in close enough proximity for the first and second magnets 24 and 68 to be drawn together using a magnetic force.

Although two separate delivery systems 14 and 16 are described, which require separate access punctures through the skin of the patient, it is contemplated that one delivery system terminating in separate distal branches may be used. For example, the separate branches may ultimately be positioned within a respective one of the first and second cardiovascular structures 220 and 222. As such, only one access puncture through the skin of the patient may be required.

Next, the second magnet delivery system 16 may be transitioned from the first delivery configuration of Fig. 1 to a first released configuration, as shown in Fig. 17, in which the second magnet 68 is released from the lumen 86 of the outer sheath 74 and positioned within the second cardiovascular structure 222. For example, this transitioning step may include moving the second magnet 68 out of magnetic engagement with the magnetized distal tip 98 of the inner tube 96 and/or moving the outer tube 72 relative to the outer sheath 74 and/or inner tube 96, as described above. According to an alternative delivery arrangement, the magnetic attraction between the first and second magnets 24 and 68 may be stronger than the attraction between the first and second magnets 24 and 68 and their respective delivery system 14, 16. This may assist in releasing or deploying the first and second magnets 24 and 68.

Once deployed or released at the designated delivery sites 232 and 240, the first and second magnets 24 and 68 may be drawn together using a magnetic force and, according to some embodiments, may be aligned with one another using alignment features of the magnets 24 and 68. Tissue of the first cardiovascular structure 220 and the second cardiovascular structure 222 may be compressed using the magnetic force between the first magnet 24 and the second magnet 68. After delivery of the first and second magnets 24 and 68, the first and second delivery catheters 26 and 70 may be removed from the patient. Over time, the compressed tissue is allowed to undergo necrosis and form a magnetic compression vascular anastomosis 250, shown in the following Figs.

To increase the speed at which the magnetic compression vascular anastomosis 250 is formed, magnets having an irregular surface profiles may be substituted for magnets 24 and 68. For example, magnets 144 and 145 having respective protrusions 146 and 147 extending from tissue engaging faces of the magnets 144 and 145, as shown in Fig. 6, may be substituted for first and second magnets 24 and 68 to increase pressure and/or damage tissue compressed therebetween to accelerate tissue necrosis. Alternatively, magnets 149 and 150 of Fig. 7 having mating profiles, such as the peak and valley profiles that are shown, may be used to accelerate tissue necrosis. Yet alternatively, a first stack of magnets 141 may be substituted for first magnet 24 and a second stack of magnets 142 may be substituted for second magnet 68 to provide a greater compressive force.

To further expedite formation of the magnetic compression vascular anastomosis 250, the magnetic compression described herein may be used in combination with a variety of other treatments. For example, radiofrequency ablation, chemical ablation, or cryoablation may be used in combination with the magnetic compression. According to additional examples, a necrotizing agent or vasoconstricting agent may additionally be used to improve tissue necrosis. Alternative assistance may be provided using vacuum or ultrasonics. These additional treatments may be applied before, during, or after placement of the first and second magnets 24 and 68 in the respective cardiovascular structures 220 and 222.

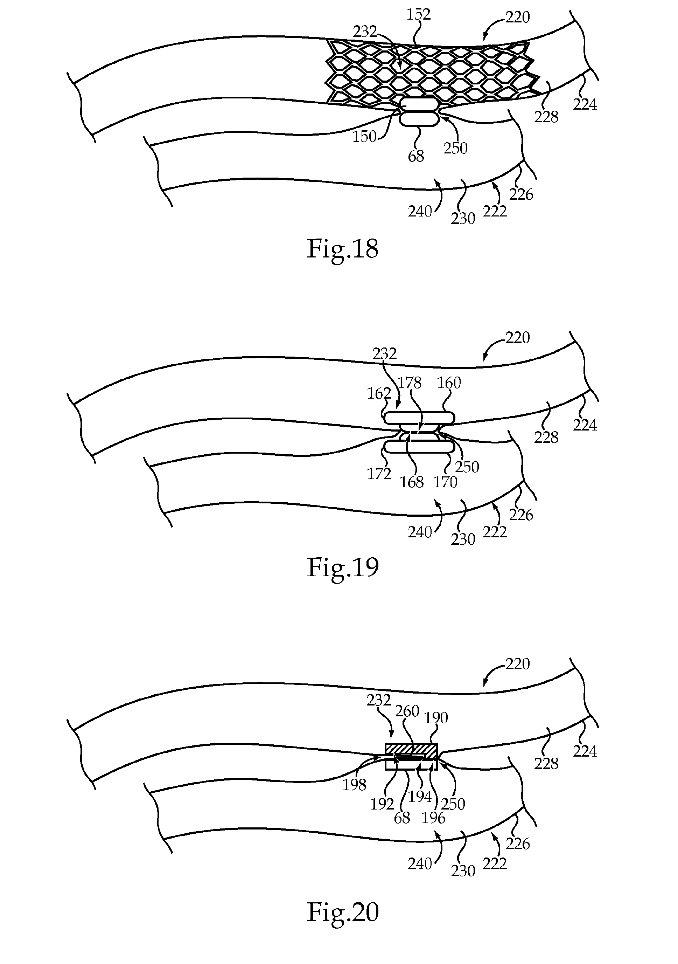

It may be desirable to anchor one or both of the first and second magnets 24 and 68 to reduce the risk of an embolism resulting from the magnets 24 and 68 becoming dislodged and carried downstream after formation of the magnetic compression vascular anastomosis 250. For example, a trap 242, or other similar mechanism, may be positioned in one or both of the first and second cardiovascular structures 220 and 222 to prevent embolization of the magnets 24 and 68. Trap 242, or an alternative capturing or anchoring mechanism, may be a radially expandable device and may be delivered simultaneously with or independent from the delivery of the magnets 24 and 68. According to another example, the anchored magnet 160 of Fig. 10 may be substituted for the first magnet 24. In particular, as shown in Fig. 18, the anchored magnet 160 may be anchored within the first cardiovascular structure 220 by radially expanding the stent 161 attached to the anchored magnet 160 within the first cardiovascular structure 220.

Alternatively, as shown in Fig. 19, the first magnet 162 and the second magnet 170, discussed above with reference to Fig. 11, may be substituted for first magnet 24 and second magnet 68, respectively. Movement of the first and second magnets 162 and 170 may be restricted after formation of the magnetic compression vascular anastomosis 250 using the radial flanges 163 and 172 of the respective magnets 162 and 170. Each of the radial flanges 163 and 172 is recessed relative to a respective one of the vascular structure engagement faces 168 and 178 of the magnets 162 and 170, as described above.

Another anchoring means is shown in Fig. 20. In particular, for example, the flap-forming magnet 190 of Fig. 12 may be substituted for the first magnet 24. As described above, the vascular structure engagement face 194 of the flap-forming magnet 190 includes the recessed area 192 that is partially surrounded by the non-recessed area 196 and extends to the edge 198 of the vascular structure engagement face 194. After formation of the magnetic compression vascular anastomosis 250, as described above, the magnets 190 and 68 will be tethered to the first and second cardiovascular structures 220 and 222 by a newly formed flap of tissue 260.

As shown in Fig. 21, one or both of the first cardiovascular structure 220 and the second cardiovascular structure 222 may be incised to remove the flap of tissue 260, the flap-forming magnet 190, and the second magnet 68. Further, the flap-forming magnet 190 and the second magnet 68 may be retrieved from a respective one of the first cardiovascular structure 220 and the second cardiovascular structure 222 after formation of the magnetic compression vascular anastomosis 250 using a retrieval catheter 270 advanced through one of the first cardiovascular structure 220 and the second cardiovascular structure 222. For example, the retrieval catheter 270 may have two lumens 272 and 274, the first lumen 272 for receiving an incising tool 276 and the second lumen 274 for receiving a tool 278, such as a wire, having a magnetized distal tip 280.

Various retrieval systems and methods are contemplated and may include the use of snaring, stent capturing, magnetic capturing, and the like. Accordingly, systems similar to the delivery systems 14 and 16, which utilize magnetic force to assist in delivering the magnets 24 and 68, may be used to retrieve the magnets 24 and 68. Alternative systems and devices for maintaining a position of the first and second magnets 24 and 68 prior to retrieval are also contemplated. For example, additional non-necrosing magnets positioned downstream relative to the first and second magnets 24 and 68 or an extracorporeal magnet may be used to reduce embolization prior to removal of the first and second magnets 24 and 68 from the cardiovascular structures 220 and 222 of the patient.

Although the magnets 24 and 68 are shown as forming the magnetic compression vascular anastomosis, the magnets 24 and 68 may be used to fuse tissue of the first and second cardiovascular structures 220 and 222 to define a perimeter of an anastomosis, while another device or system is used to form the actual openings through the cardiovascular structures 220 and 222. For example, the opening may be created using ablation, puncturing, cutting, balloon dilation, or the like.

Conventionally, shunts in the cardiovascular system are formed using invasive surgical procedures. The system and method disclosed herein provides a non-invasive means for forming a shunt in the cardiovascular system. In particular, a magnetic compression vascular anastomosis may be formed by introducing magnets or magnet assemblies into respective cardiovascular structures using an endovascular delivery system. As such, a cardiovascular shunt may be formed, according to the present disclosure, in a manner that is less invasive, with fewer accompanying risks and a faster recovery time, as compared to conventional procedures.

It should be understood that the above description is intended for illustrative purposes only, and is not intended to limit the scope of the present disclosure in any way. Thus, those skilled in the art will appreciate that other aspects of the disclosure can be obtained from a study of the drawings, the disclosure and the appended claims.

The foregoing disclosure may be summarised as follows.

An endovascular delivery system for forming a magnetic compression vascular anastomosis includes a first compressing element and a first catheter configured for advancement into a first cardiovascular structure. In one configuration, the first compressing element is removably secured within the first catheter and, in another configuration, the first compressing element is released from the first catheter and positioned within the first cardiovascular structure. The endovascular delivery system also includes a second compressing element and a second catheter configured for advancement into a second cardiovascular structure. In one configuration, the second compressing element is removably secured within the second catheter and, in another configuration, the second compressing element is released from the second catheter and positioned within the second cardiovascular structure. The first and second compressing elements have an anastomosis forming configuration in which the compressing elements compress tissue of the first and second cardiovascular structures using magnetic force.

## Claims

1. An endovascular delivery system for forming a magnetic compression vascular anastomosis, comprising:
a first magnet delivery system including a first compressing element and a first delivery catheter configured for advancement into a first cardiovascular structure, wherein the first magnet delivery system has a delivery configuration in which the first compressing element is removably secured within a lumen of the first delivery catheter, and a released configuration in which the first compressing element is released from the lumen of the first delivery catheter and positioned within the first cardiovascular structure; and
a second magnet delivery system including a second compressing element and a second delivery catheter configured for advancement into a second cardiovascular structure, wherein the second magnet delivery system has a delivery configuration in which the second compressing element is removably secured within a lumen of the second delivery catheter, and a released configuration in which the second compressing element is released from the lumen of the second delivery catheter and positioned within the second cardiovascular structure;
wherein the first compressing element and the second compressing element have an anastomosis forming configuration in which the first compressing element and the second compressing element compress tissue of the first cardiovascular structure and the second cardiovascular structure using a magnetic force between the first compressing element and the second compressing element.

2. The endovascular delivery system of claim 1, wherein at least the first compressing element is a magnet assembly including a plurality of magnets, wherein the magnet assembly has a delivery configuration in which the plurality of magnets are sequentially disposed within the lumen of the first delivery catheter, and an assembled configuration in which the plurality of magnets are released from the lumen of the first delivery catheter and secured to one another magnetically or mechanically.

3. The endovascular delivery system of claim 1 or claim 2, wherein each of the first compressing element and the second compressing element includes an irregular surface profile configured to engage tissue of a respective one of the first cardiovascular structure and the second cardiovascular structure in the anastomosis forming configuration.

4. The endovascular delivery system of any one of the preceding claims, wherein each of the first compressing element and the second compressing element includes an RFID chip configured to generate a signal indicative of formation of the magnetic compression vascular anastomosis responsive to contact between the first compressing element and the second compressing element.

5. The endovascular delivery system of any one of the preceding claims, wherein at least the first compressing element is an anchored magnet attached to a radially expandable prosthetic implant configured to anchor the anchored magnet within the first cardiovascular structure.

6. The endovascular delivery system of any one of the preceding claims, wherein each of the first compressing element and the second compressing element includes a radial flange recessed relative to a vascular structure engagement face of a respective one of the first compressing element and the second compressing element.

7. The endovascular delivery system any one of the preceding claims, wherein at least the first compressing element is a flap-forming magnet having a recessed area along a vascular structure engagement face of the flap-forming magnet, wherein the recessed area is partially surrounded by a non-recessed area of the vascular structure engagement face and extends to an edge of the vascular structure engagement face.

8. The endovascular delivery system of any one of the preceding claims, wherein the first compressing element is removably secured within the lumen of the first delivery catheter using a magnetic force.

9. The endovascular delivery system of claim 8, wherein the first delivery catheter includes an inner tube having a magnetized or magnetically attractive distal tip telescopically received within an outer tube, wherein, in the delivery configuration of the first magnet delivery system, the first compressing element is distally disposed relative to the outer tube, and is removably secured to the inner tube using the magnetic force between the first compressing element and the magnetized or magnetically attractive distal tip of the inner tube.

10. The endovascular delivery system of any one of the preceding claims, further including a trap positioned within at least one of the first cardiovascular structure and the second cardiovascular structure downstream relative to the magnetic compression vascular anastomosis and configured to capture the first compressing element and the second compressing element after formation of the magnetic compression vascular anastomosis.

11. The endovascular delivery system of any one of the preceding claims wherein the first magnet delivery system has a pre-release configuration in which the first compressing element is positioned outside the lumen of the first delivery catheter and mechanically connected with a mechanical connection to an inner member that is telescopically received in the first delivery catheter.

12. The endovascular delivery system of claim 11 wherein the mechanical connection includes a suture extending through a hole defined by the first compressing element.

13. The endovascular delivery system of any one of the preceding claims wherein the first compressing element has a length to diameter ratio greater than one, and a magnetic pole axis oriented perpendicular to a long axis.

14. The endovascular delivery system of any one of the preceding claims wherein a length of the first compressing element is greater than a diameter of the lumen of the first delivery catheter.

15. The endovascular delivery system of any one of the preceding claims wherein first compressing element has a male surface feature sized to be received in a female surface feature of the second compressing element and preferably wherein the female surface feature is at least partially defined by a non-magnetic jacket.
